# FASCICULE DE BREVET EUROPEEN

(11) **EP 0 888 133 B1**
(45) Date de publication et mention de la délivrance du brevet: **18.06.2003**
(21) Numéro de dépôt: 97947180.2
(22) Date de dépôt: 16.12.1997
(51) Int. Cl.: A61L 2/08

(54) **INSTALLATION DE STERILISATION DE PRODUITS MEDICAUX PAR IRRADIATION**
STERILISATIONANLAGE VON MEDIZINISCHEN PRODUKTEN DURCH BESTRAHLUNG
SYSTEM FOR STERILIZING MEDICINAL PRODUCTS BY RADIATION PROCESSING

(30) Priorité: 17.12.1996 IT TO961037
(43) Date de publication de la demande: 07.01.1999
(60) Demande divisionnaire: 03075810.6
(73) Titulaire: GAMBRO HOSPAL (Schweiz) AG, 4001 Basel (CH)
(72) Inventeur: ALBORESI, Luigi, I-41100 Modena (IT); SANTI, Marco, I-41100 Modena (IT)
(74) Mandataire: Lejeune, Daniel
(86) Numéro de dépôt international: IB9701570
(87) Numéro de publication internationale: WO98026805

(56) Documents cités:
- GB-A- 764 337
- GB-A- 1 525 484
- US-A- 3 264 473

## Description

La présente invention concerne une installation de stérilisation de produits médicaux par irradiation.

Il est connu que les canalisations pour circulation extracorporelle de sang dites lignes à sang utilisées pour les traitements extracorporels de sang comme l'hémodialyse sont soumis à un procédé de stérilisation visant à assurer l'élimination complète des germes. Actuellement, cette stérilisation est effectuée de différentes manières, par exemple par irradiation par rayons gamma. Toutefois, cette technique présente divers inconvénients, liés au caractère dangereux du matériau utilisé pour la production des radiations, au coût du blindage des équipements d'irradiation et à la difficulté d'obtenir les autorisations officielles nécessaires à leur utilisation en raison de l'attention croissante portée à la pollution de l'environnement. Par ailleurs, la stérilisation à la vapeur à haute température convient mal aux lignes à sang en PVC car, pour assurer une stérilisation adéquate, il est nécessaire d'utiliser une température voisine du point de ramollissement du PVC, ce qui entraîne un risque de détérioration des produits.

Actuellement, pour d'autres types de produits, on utilise d'autres procédés d'irradiation, tels que l'irradiation par rayons bêta, qui ne causent pas les problèmes mentionnés plus haut. La stérilisation par rayons bêta est, par exemple, utilisée pour le traitement de produits comestibles ou de produits médicaux à caractère non critique, avec de bons résultats.

Le document GB-A-1 525 484 décrit une installation d'irradiation et prévoit d'irradier des récipients et leurs couvercles simultanément avec des dosages différents.

Toutefois, dans le cas des lignes à sang en PVC, ce traitement de stérilisation n'a pas encore été utilisé à grande échelle en raison du caractère critique du matériau et des exigences élevées de stérilisation requises pour une telle application. En fait, d'une part, les caractéristiques chimiques et physiques du PVC sont extrêmement sensibles aux doses d'irradiation et imposent des limites supérieures strictes aux doses utilisables et, d'autre part, la nécessité d'une stérilisation suffisante impose des limites inférieures à ces doses. Par ailleurs, les installations d'irradiation qui n'ont pas été conçues spécifiquement (telles que les installations de service pour des tiers) ne permettent pas de garantir une stérilisation suffisante de l'ensemble du produit sans qu'une partie du produit ne soit soumis à une exposition excessive. A cela vient s'ajouter que le prix unitaire réduit du produit ne permet pas d'utiliser des techniques de contrôle et de gestion coûteuses si l'on veut maintenir la compétitivité des lignes à sang stérilisées par rayons bêta par rapport aux lignes stérilisées par les procédés classiques.

Le but de la présente invention consiste à réaliser une installation de stérilisation qui permette de stériliser efficacement et économiquement des produits fragiles tels que des lignes à sang en PVC sans les détériorer d'aucune façon.

Pour atteindre ce but on prévoit, conformément à l'invention, une installation de stérilisation de produits médicaux par irradiation, comprenant:
- au moins une station de chargement de produits,
- au moins une station de déchargement de produits,
- une station d'irradiation située à l'intérieur d'un caisson ayant des parois aptes à arrêter les rayonnements stérilisants,
- des moyens de convoyage pour acheminer les produits à stériliser de la station de chargement à la station d'irradiation et les produits stérilisés de la station d'irradiation à la station de déchargement, les moyens de convoyage comprenant des moyens de regroupement pour regrouper les produits à stériliser à proximité de la station d'irradiation, les moyens de regroupement comprenant un premier convoyeur et un deuxième convoyeur, le deuxième convoyeur étant situé immédiatement en aval du premier convoyeur par rapport à la direction de convoyage des produits et ayant une vitesse de convoyage inférieure à la vitesse de convoyage du premier convoyeur.

Selon une caractéristique de l'invention, le premier et le deuxième convoyeurs comprennent des organes d'entraînement rétractables pour accrocher les produits et assujettir rigoureusement leur déplacement à celui du convoyeur correspondant.

Selon une caractéristique de l'invention, le deuxième convoyeur comprend deux demi convoyeurs séparés par un interstice disposé dans une zone vers laquelle la station d'irradiation émet les rayonnements stérilisants.

Selon une caractéristique de l'invention, l'installation comprend un troisième convoyeur situé immédiatement en aval du deuxième convoyeur par rapport à la direction de convoyage des produits, le troisième convoyeur ayant une vitesse de convoyage supérieure à la vitesse de convoyage du deuxième convoyeur.

Selon une caractéristique de l'invention, les moyens de convoyage comprennent:
- des sections de convoyage superposées, les sections de convoyage situées à un premier niveau acheminant les produits à stériliser de la station de chargement à la station d'irradiation et les sections de convoyage situées à un second niveau acheminant les produits stérilisés de la station d'irradiation à la station de déchargement.
- un transporteur vertical pour transférer les produits d'un niveau de convoyage à l'autre niveau de convoyage.

Selon une caractéristique de l'invention, l'installation comprend des moyens de détection de produits, disposés le long des moyens de convoyage, et des moyens de commande et de contrôle reliés aux moyens de détection pour suivre le cheminement des produits sur les moyens de convoyage.

Selon une caractéristique de l'invention, l'installation comprend une pluralité de plateaux destinés à supporter les produits sur les moyens de convoyage, chaque plateau étant muni d'un code d'identification, les moyens de détection de produits comprenant des moyens de lecture de code aptes à lire le code apposé sur chaque plateau.

Selon une caractéristique de l'invention, les moyens de détection de produits comprennent des moyens de lecture de code aptes à lire un code d'identification apposé sur chacun des produits disposé sur chaque plateau et l'unité de commande et de contrôle est prévue pour associer et mettre en mémoire le code d'identification de chaque produit et le code d'identification du plateau qui supporte ce produit.

Selon une caractéristique de l'invention, les moyens de détection de produits comprennent des moyens de détection de fin de course mécaniques disposés à l'intérieur du caisson pour détecter la position des plateaux à l'intérieur du caisson et transmettre un signal correspondant à l'unité de commande et de contrôle.

Selon une caractéristique de l'invention, l'installation comporte des moyens de mesure de la vitesse du deuxième convoyeur reliés aux moyens de commande et de contrôle, et les moyens de commande et de contrôle sont prévus pour ajuster l'irradiation émise par la station d'irradiation en fonction des variations de la vitesse du convoyeur de façon que les produits reçoivent une dose d'irradiation prédéterminée.

D'autres avantages et caractéristiques de l'invention apparaîtront à la lecture de la description qui suit. On se reportera aux dessins annexés sur lesquels:
la figure 1 représente une vue de dessus de l'ensemble de la l'installation de stérilisation selon l'invention;
la figure 2 représente schématiquement une ligne à sang à usage unique;
la figure 3 représente une vue en perspective de deux boîtes contenant chacune plusieurs lignes à sang;
la figure 4 représente une vue en perspective d'un plateau pour le transport des deux boîtes de la figure 3;
la figure 5 représente un détail agrandi de la figure 1,
la figure 6 représente une vue latérale d'une partie de l'installation de la figure 1;
la figure 7 représente un détail agrandi de la figure 6;
la figure 8 représente un autre détail agrandi de la figure 1;

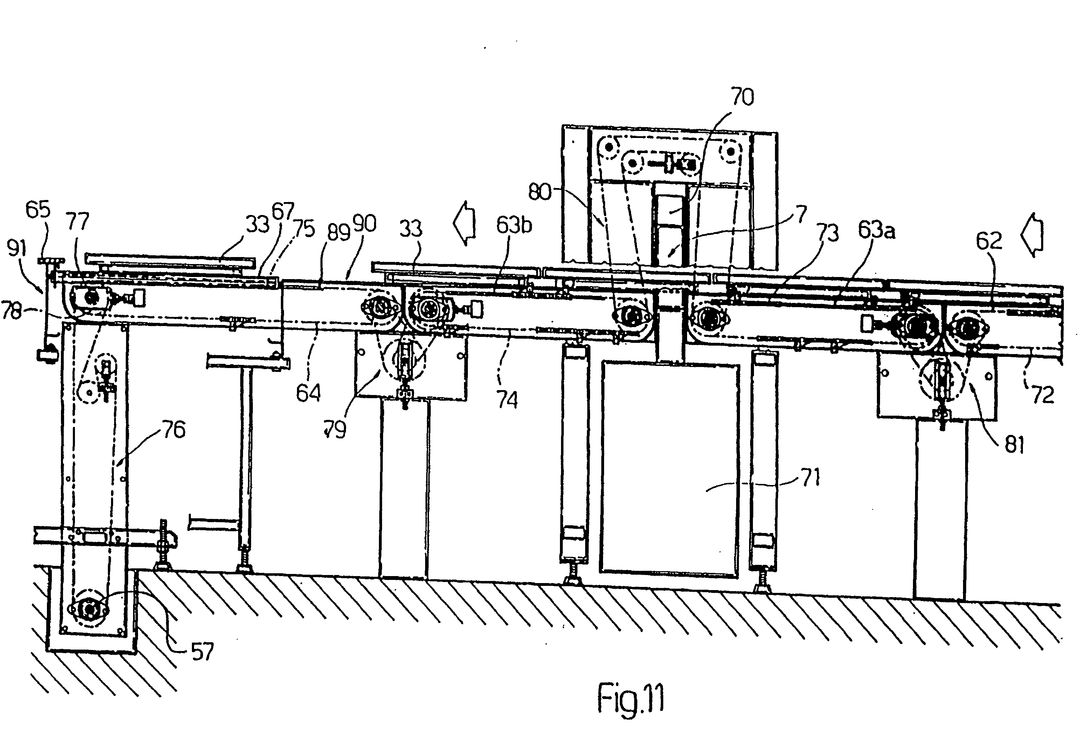

la figure 9 représente une coupe transversale de l'installation prise suivant la ligne IX-IX de la figure 1;
la figure 10 représente un autre détail agrandi de la figure 1;
la figure 11 représente une vue latérale d'une partie de l'installation de la figure 1;
la figure 12 représente un détail des convoyeurs de la figure 11;
les figures 13 et 14 représentent des vues latérales, dans deux positions différentes, des organes d'entraînement des convoyeurs de la figure 11;
la figure 15 représente une coupe transversale d'une partie des convoyeurs de la figure 11;
la figure 16 représente une coupe transversale de l'installation, prise suivant la ligne XVI-XVI de la figure 1; et
les figures 17a et 17b représentent un schéma synoptique relatif aux fonctions de commande de l'unité de commande et de contrôle.

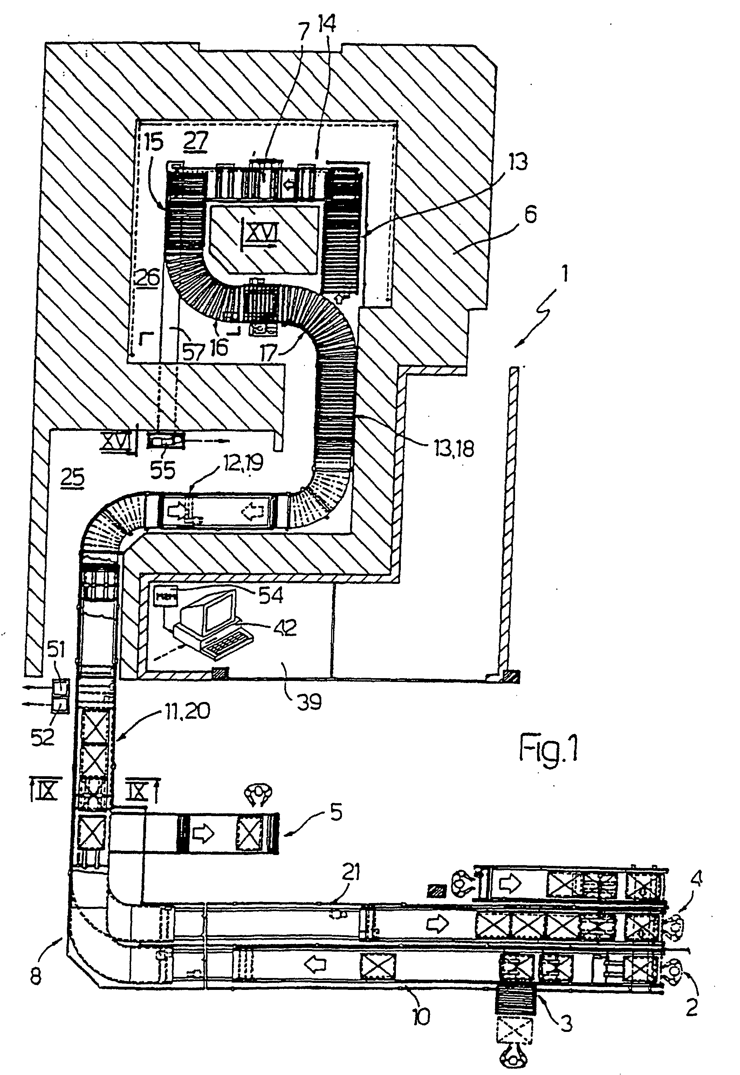

En se référant à la figure 1, l'installation de stérilisation 1 comprend une station de chargement principale 2, une station de chargement auxiliaire 3, une station de déchargement principale 4, une station de déchargement auxiliaire 5, un caisson ou bunker 6, une station d'irradiation 7 disposée à l'intérieur du caisson 6 et des moyens de convoyage 8 reliant la station de chargement 2 à la station de déchargement 4.

Les moyens de convoyage 8 comportent une pluralité de convoyeurs, de préférence du type à rouleaux, avec des sections de convoyage de produits à stériliser et des sections de convoyage de produits stérilisés qui sont superposées. En particulier, les moyens de convoyage 8 comportent les sections successives suivantes:
- une première section 10 disposée dans le prolongement de la station de chargement principale 2,
- une deuxième section 11 perpendiculaire à la première section 10,
- une troisième section 12 perpendiculaire à la deuxième section 11 et s'étendant dans une direction opposée à celle de la première section 10,
- une quatrième section 13 perpendiculaire à la troisième section 12 et s'étendant dans la même direction que la deuxième section 11,
- une cinquième section 14, traversant la station d'irradiation 7, perpendiculaire à la quatrième section 13 et s'étendant dans une direction opposée à celle de la troisième section 12,
- une sixième section 15 perpendiculaire à la cinquième section 14 et s'étendant dans une direction opposée à celle de la quatrième section 13,
- une septième section 16 de forme courbe ayant sa concavité tournée vers la station d'irradiation 7,
- une huitième section 17 de forme courbe ayant sa concavité tournée à l'opposé de la station d'irradiation 7, cette huitième section 17 étant dans un plan situé au-dessous d'un plan parallèle contenant les sept sections précédentes,
- une neuvième section 18, une dixième section 19 et une onzième section 20, s'étendant respectivement au dessous de la quatrième section 13, de la troisième section 12 et de la deuxième section 11, et
- une douzième section 21 parallèle à la première section 10 et prolongée par la station de déchargement principale 4.

En d'autres termes, les sections 13 à 17 forment un anneau à l'intérieur du caisson 6, et sont précédées et suivies par des sections superposées pour l'acheminement des boîtes de produits à irradier et de boîtes irradiées.

Toutes les sections de convoyage forment entre elles un angle, à l'exception des sections 13 à 15, sont raccordées entre elles par des portions courbes.

Le caisson 6 est réalisée en maçonnerie et il délimite trois zones qui communiquent entre elles:
- une première zone 25 d'entrée et de sortie, contenant une partie des sections 11, 20, les sections 12, 19 et une partie des sections 13, 18,
- une seconde zone 26, intermédiaire, contenant une partie des sections 13, 18 et les sections 15 à 17, et
- une troisième zone 27 contenant la section 14 et la station d'irradiation 7.

La troisième zone 27 ne contient pas d'appareillages électriques ou électroniques qui pourraient être endommagés par les radiations ionisantes produites dans la station d'irradiation 7. Par ailleurs, les organes mécaniques qui s'y trouvent sont réalisés à partir de matériaux très résistants à l'ionisation, tels que l'acier inoxydable, et lubrifiés par des produits spécifiques.

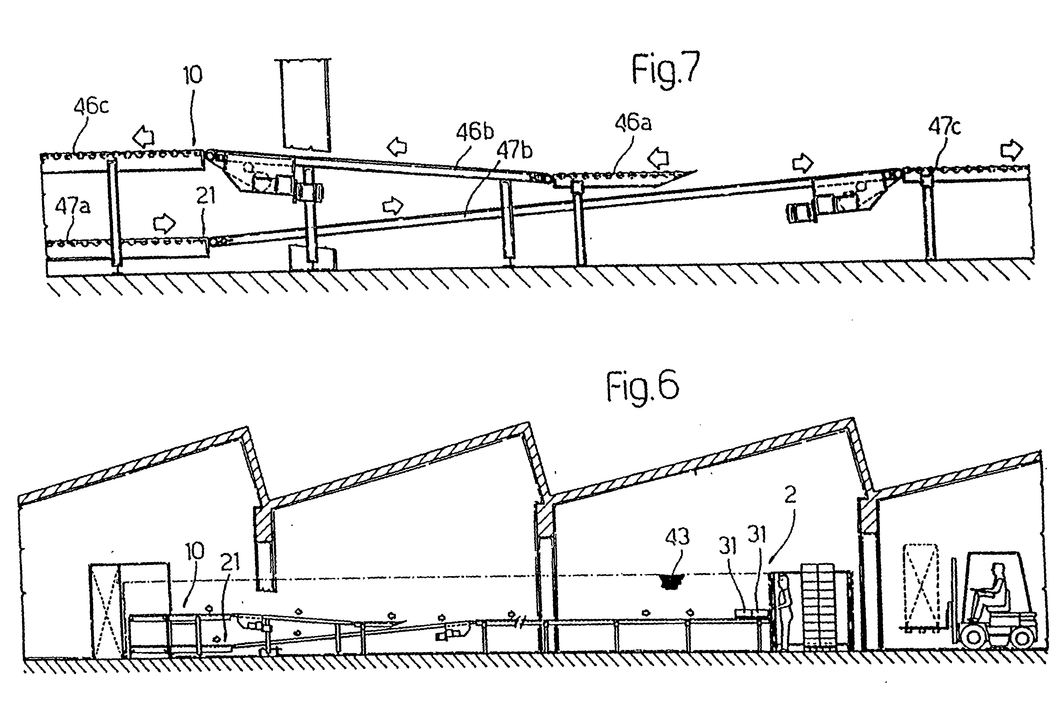

Les sections des moyens de convoyage situées à l'extérieur du caisson 6 sont enfermées dans une cage en treillis métallique 43 en interdisant l'accès (voir figure 6).

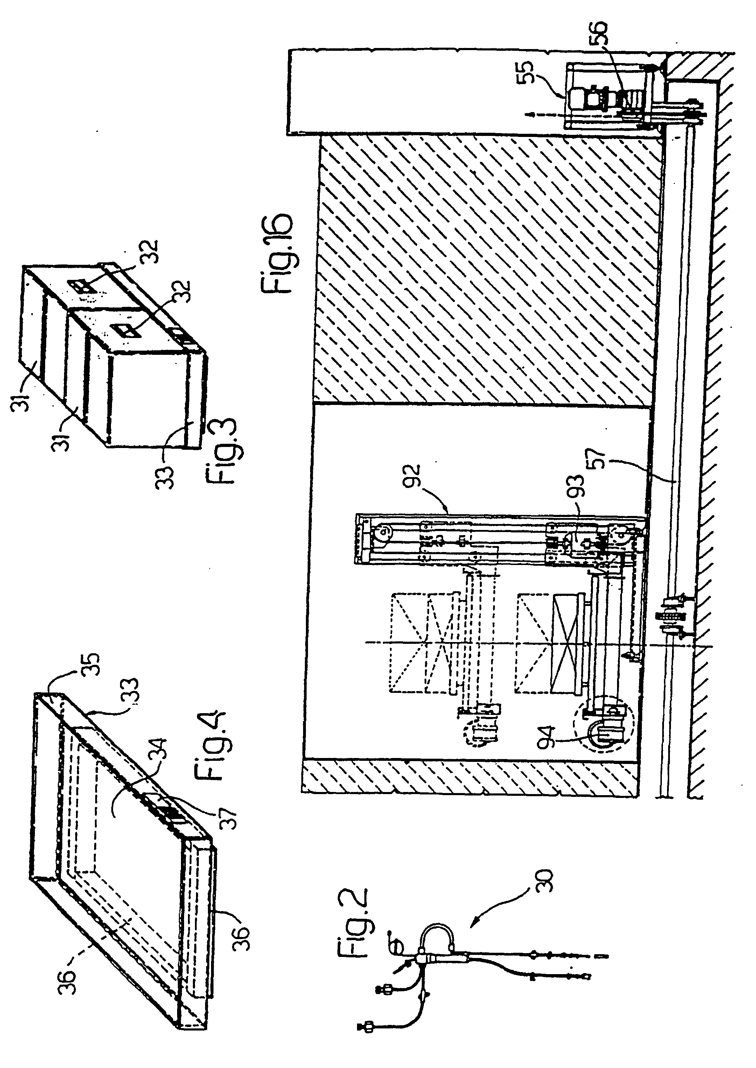

L'installation 1 est destinée à la stérilisation de lignes à sang, par exemple du type illustré sur la figure 2 et repéré par le chiffre de référence 30. Plusieurs lignes à sang 30 sont disposées dans une boîte 31 (figure 3) pourvue d'une étiquette de boîte 32 portant un code d'identification de produit (par exemple, un code à barres). Deux boîtes 31 sont disposées côte à côte sur un plateau rectangulaire 33 (voir en particulier la figure 4), de préférence métallique, qui comprend un fond 34 plat muni d'un rebord périphérique 35. A la face inférieure du fond 34 est fixé un cadre 36 dont les côtés sont en léger retrait par rapport aux rebords correspondants du fond 34. Une étiquette de plateau 37 est collée sur le rebord 35 du plateau 34, laquelle porte un code d'identification (typiquement, un numéro spécifique au plateau ou un code à barres).

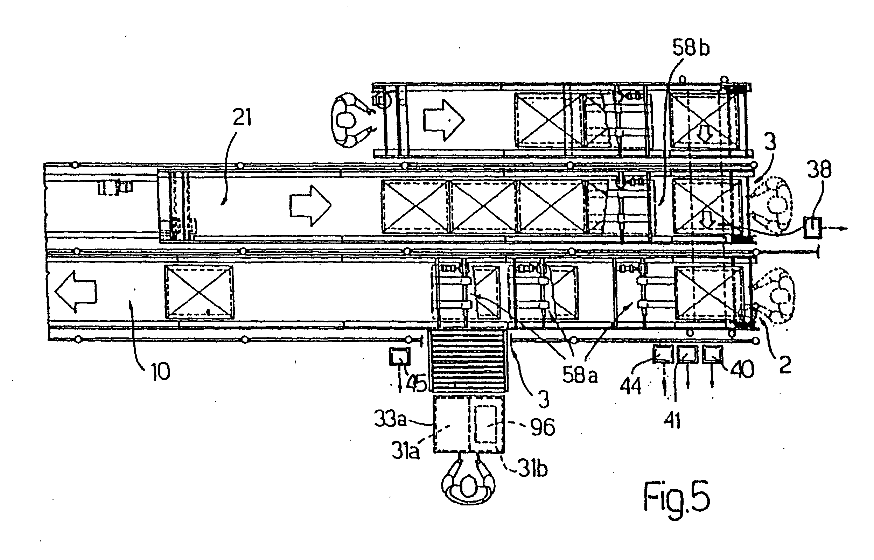

A la station de chargement principale 2 (voir figure 5), les plateaux 33 sont chargés de manière que les deux boîtes 31 avancent l'une derrière l'autre sur les moyens de convoyage. La station de chargement principale 2 comprend un groupe de lecteurs de codes 40, 41, 44 de type optique qui sont disposés latéralement au convoyeur et orientés de manière que chacun d'eux puisse lire le code d'identification des deux boîtes et le code d'identification du plateau sur les étiquettes respectives 32, 37 et émettre des signaux d'identification correspondants. Les lecteurs de codes 40, 41, 44 ainsi que les autres lecteurs de codes mentionnés dans la suite sont reliés à une unité de commande et de contrôle 42 (voir figure 1) située dans un local 39 contigu au caisson 6. L'unité de commande et de contrôle 42 est reliée à une mémoire 54 et, le cas échéant à une unité de traitement située à un emplacement approprié le long des moyens de convoyage 8 ainsi qu'à des dispositifs de signalisation optique et/ou sonore, non représentés de façon détaillée.

Une station de chargement auxiliaire 3 est disposée latéralement à la première section 10 des moyens de convoyage. Cette station auxiliaire 3 est munie d'un lecteur de code 45 pour l'étiquette d'un plateau, destiné à émettre un signal de code correspondant.

Comme illustré sur les figures 5 à 7, la première et la douzième sections de convoyage 10 et 21 comportent chacune trois convoyeurs différents 46a, 46b, 46c, respectivement, 47a, 47b, 47c, chacun d'eux définissant un trajet initial, un trajet intermédiaire et un trajet final dans la direction de convoyage respective. La station de chargement principale 2 et la station de déchargement principale 4 étant disposées côte à côte, les convoyeurs 46a et 47c sont au même niveau. Les convoyeurs intermédiaires 46b, 47b définissent des rampes toutes deux ascendantes dans la direction d'acheminement des plateaux 33. Le convoyeur 47a est disposé latéralement au convoyeur 46c, à un niveau inférieur. Les convoyeurs 46a et 47c sont munis de dispositifs de blocage 58a, 58b, illustrés schématiquement et non décrits de façon détaillée. La station de déchargement principale 4 comporte en outre un lecteur de code 38 disposé de manière à permettre la lecture de l'étiquette 37 du plateau 33 arrivant en fin de trajet.

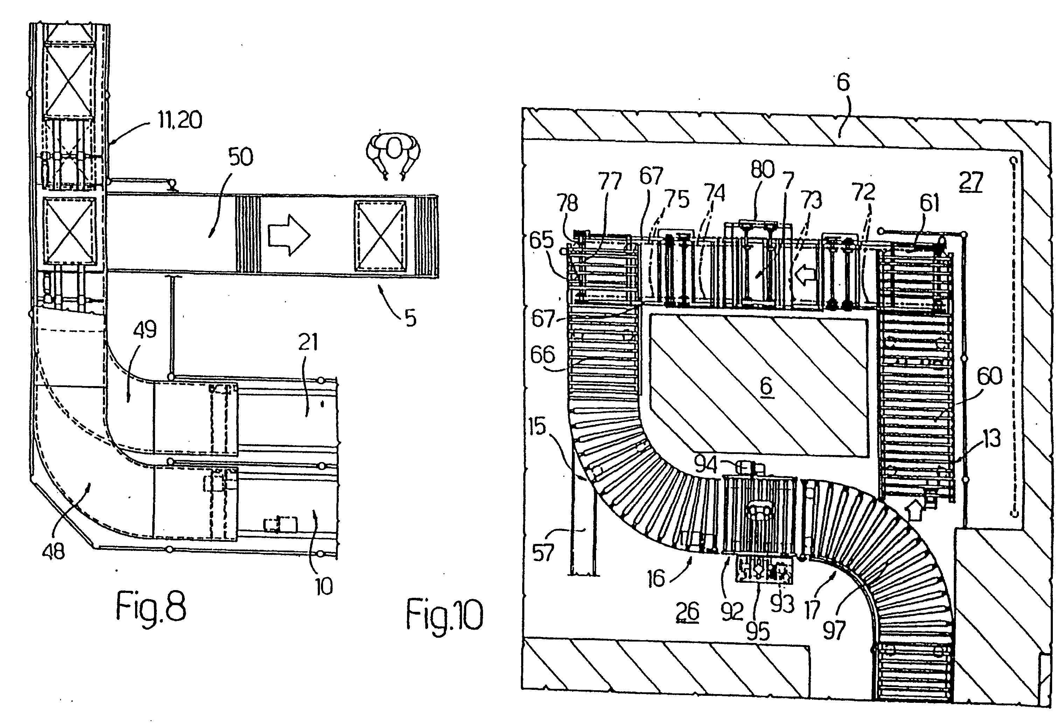

Les sections 10 et 21 sont raccordées aux sections contiguës 11, 20 par l'intermédiaire de portion de raccordement courbes, respectivement 48, 49 (voir figure 8) de façon que les sections 11 et 20 soient exactement superposées.

Comme représenté de façon détaillée sur la figure 8, la section de convoyage 20 est reliée à la station de déchargement auxiliaire 5 par l'intermédiaire d'une section de jonction 50 perpendiculaire à la section de convoyage 20. Chaque section de convoyage 11, 20 est équipée d'un lecteur de codes respectif 51, 52 (figure 1).

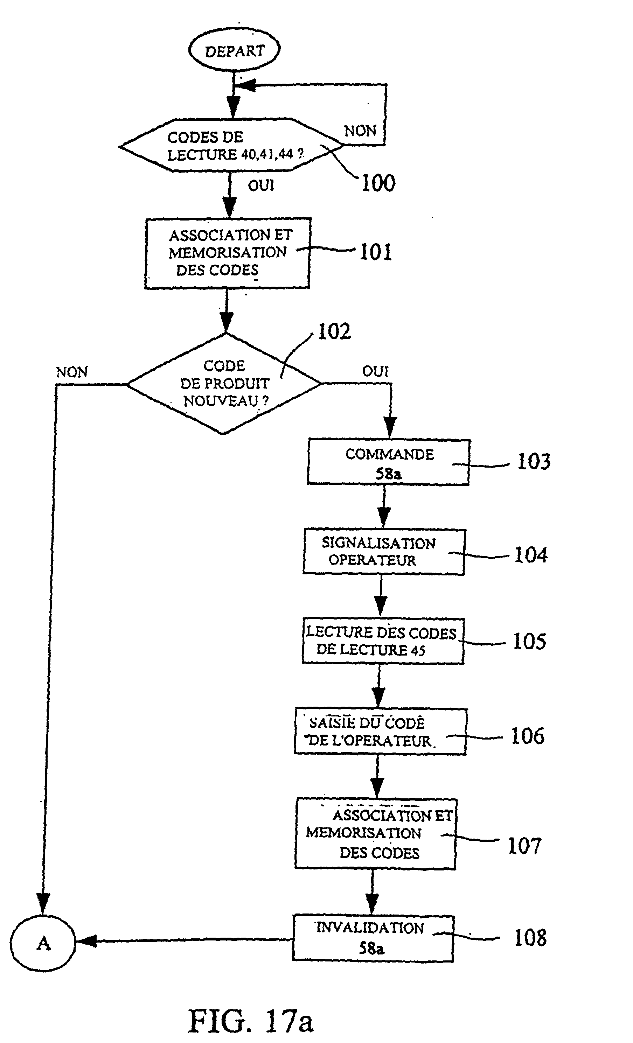

A l'intérieur du caisson 6, l'entraînement des convoyeurs définissant la section 14 est assuré par un moteur unique 55 (voir figures 1 et 15) disposé dans la zone d'entrée et de sortie 25, et donc à l'extérieur de la zone critique 27. Le moteur 55 est relié aux convoyeurs de la section 14 par un arbre 57 traversant une paroi du caisson 6. Le moteur 55 comprend une dynamo tachymétrique 56, qui en contrôle la vitesse avec précision et émet des signaux qui sont fournis à l'unité de commande et de contrôle 42 pour vérifier le fonctionnement correct du système de convoyeurs et, éventuellement, l'adéquation des paramètres du procédé.

La section de convoyage 13, qui s'étend de la zone d'entrée et de sortie 25 à la zone de stérilisation 27, comprend un convoyeur 60 à rouleaux en acier inoxydable et, à son extrémité contiguë à la section 14, un détecteur de fin de course 61, de type à bras mécanique, représenté de façon schématique sur la figure 10. Le détecteur de fin de course 61 est relié à l'unité de commande et de contrôle 42 par l'intermédiaire d'une tringlerie et de transducteurs.

La section 14 comprend trois convoyeurs à chaîne 62, 63, 64. Les convoyeurs d'extrémité, 62 et 64, ont une vitesse d'acheminement élevée, tandis que la vitesse du convoyeur central 63 est réduite. Des rails de guidage 67 sont disposés de chaque côté de la section 14, à l'extérieur des convoyeurs à chaîne 62 à 64, pour supporter et guider les plateaux 33. Un détecteur mécanique à bras (non représenté) est monté au niveau du convoyeur central 63 pour détecter la présence des plateaux 33. Un détecteur de fin de course 65 est monté à l'extrémité du convoyeur 64 contiguë à la section de convoyage 15 pour détecter l'arrivée d'un plateau 33 et déclencher le fonctionnement du convoyeur à rouleaux 66 de la section de convoyage 15.

Comme on peut le voir sur la figure 11, le convoyeur central 63 comprend deux demi convoyeurs 63a, 63b séparés par un interstice situé dans la station d'irradiation 7. Cette station comprend un accélérateur de particules 70 muni d'une tête de balayage, non représentée, qui est disposée au dessus du trajet des produits à stériliser, à l'aplomb de l'interstice séparant les deux demi convoyeurs 63a, 63b, et un puits d'absorption 71 disposé à l'aplomb de une tête de balayage, au dessous du niveau des convoyeurs 63a, 63b, pour absorber et dissiper l'énergie excédentaire des radiations bêta.

Comme cela est représenté schématiquement sur les figures 10 et 11, le convoyeur 62 est constitué par une paire de chaînes 72, le demi convoyeur 63a est constitué par une paire de chaînes 73, le demi convoyeur 63b est constitué par une paire de chaînes 74 et le convoyeur 64 est constitué par une paire de chaînes 75. Pour l'actionnement de ces convoyeurs, l'arbre 57 entraîne la paire de chaînes 75 par l'intermédiaire d'une transmission à chaîne 76, d'un arbre moteur 77 et d'une roue dentée 78; la paire de chaînes 75 entraîne la paire de chaînes 74 par l'intermédiaire d'un train réducteur 79 situé au dessous des convoyeurs; la paire de chaînes 74 entraîne la paire de chaînes 73 par l'intermédiaire d'un mécanisme de renvoi 80 disposé dessus des convoyeurs; et la paire de chaînes 73 entraîne la paire de chaînes 72 par l'intermédiaire d'un train multiplicateur 81 situé au dessous des convoyeurs.

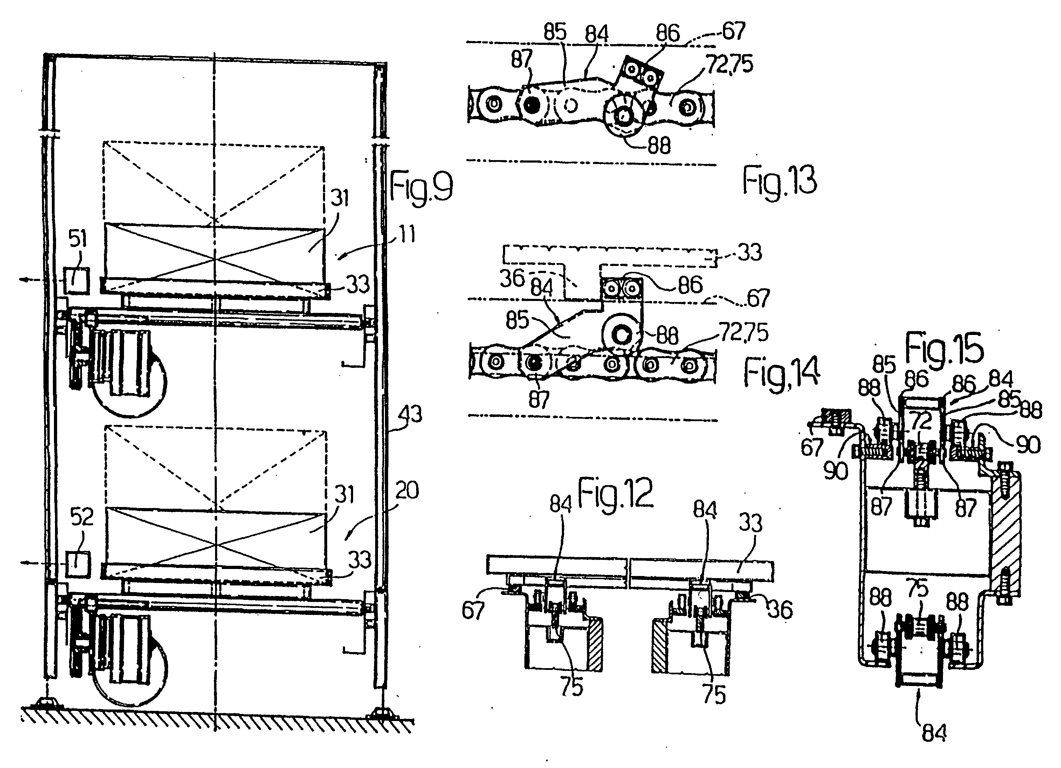

Comme on peut le voir sur les figures 12 à 16, les chaînes 72 à 75 comprennent des paires d'organes d'entraînement 84 alignés (figure 12). Chaque organe d'entraînement 84 est constitué par une paire d'éléments 85 en forme d'équerre connectés rigidement l'un à l'autre à une première extrémité 86 et connectés de façon pivotante à la chaîne à une seconde extrémité 87 (figure 15). Un rouleau 88 est monté pivotant sur la face extérieure de chaque élément 85 en forme d'équerre, au niveau de sa partie coudée. Dans des sections prédéterminées de la portion supérieure de chaque chaîne 72 à 75, les rouleaux 88 d'un organe d'entraînement 84 viennent engager des guides rectilignes parallèles correspondants 90 (voir figures 11 et 15) ce qui a pour effet de faire passer l'organe d'entraînement 84 d'une position rétractée où il est au dessous du niveau des rails de guidage 67 des plateaux 33 (voir figure 13), à une position saillante (voir figure 14) où il peut s'engager dans le cadre 36 fixé sous chaque plateau 33. La position des organes d'entraînement 84 sur chaque chaîne 72 à 75, la synchronisation des chaînes et la longueur et le disposition des guides rectilignes 90 sont calculés de manière adéquate pour assurer l'entraînement de chaque plateau 33 par les chaînes 72 à 75 au moment voulu ainsi que le groupement correct des plateaux 33, comme décrit ci-dessous d'une façon plus détaillée.

Sur la figure 11 on a représenté en outre schématiquement le détecteur de fin de course 65 et la tringlerie correspondante 91 qui sert à actionner le convoyeur à rouleaux 66 des sections de convoyage 15 et 16. La liaison entre les sections de convoyage 16 et 17 qui ne sont pas au même niveau est assuré par un transporteur vertical 92 (voir figure 10) qui est prévu pour prélever un plateau 33 arrivant à l'extrémité du convoyeur à rouleaux 66 et le transférer sur le convoyeur à rouleaux 97 des sections de convoyage 17 et 18. Le transporteur vertical 92, qui est disposé dans la zone 26 du caisson 6, comprend un moteur 93 pour l'acheminement vertical des plateaux 33, un moteur 94 pour leur acheminement horizontal ainsi que des moyens de commande 95 spécifiques pour assurer la synchronisation avec les convoyeurs rouleaux 66 et 97.

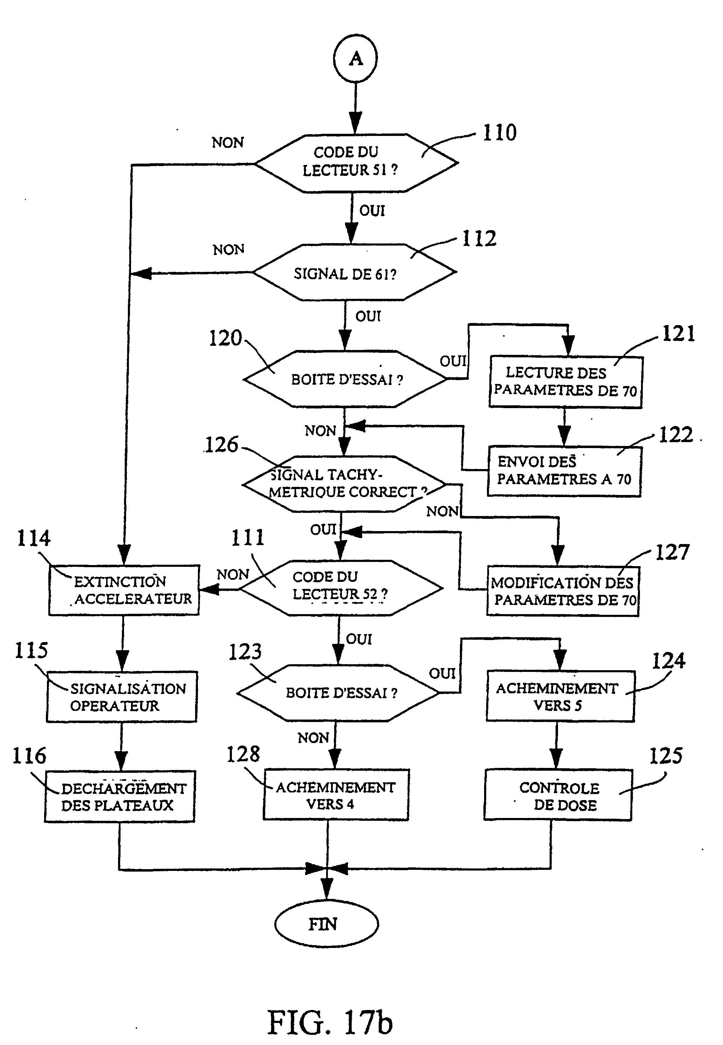

L'installation qui vient d'être décrite fonctionne de la façon suivante. A la station de chargement principale 2, l'opérateur place deux boîtes 31 contenant des lignes à sang 30, l'une devant l'autre, sur un plateau 33. Comme illustré sur l'organigramme des figures 17a, 17b, les lecteurs de code 40, 41 et 44 lisent les étiquettes 32 et 37 des boîtes 31 et du plateau 33 (bloc 100) et émettent les signaux correspondants qui sont transmis à l'unité de commande et de contrôle 42, laquelle associe (bloc 101) les données relatives aux produits, lues sur l'étiquette de la boîte 31, au numéro d'identification du plateau, lu sur l'étiquette du plateau 33. Pendant toute la durée du séjour des boîtes 31 à l'intérieur de l'installation 1, celles-ci sont identifiées et suivies sur les moyens de convoyage uniquement à partir du code d'identification du plateau correspondant 33, auquel elles sont associées sans risque d'échange ni d'erreur : on rappelle que l'ensemble des moyens de convoyage est inaccessible aux opérateurs puisqu'il est isolé de l'extérieur par la cage en treillis métallique 43 et par le caisson 6.

L'unité de commande et de contrôle 42 vérifie, en outre, si le code de produit lu sur l'étiquette des boîtes 31 correspond à celui des produits chargés et traités précédemment (bloc 102); dans la négative (produits différents nécessitant une variation des paramètres de traitement, tels que l'intensité de l'irradiation produite par l'accélérateur 70 ou la vitesse d'acheminement du convoyeur 14), l'unité de commande 42 actionne les dispositifs de blocage 58a, ce qui provoque l'arrêt des plateaux 33 déjà introduits dans la station de chargement principale 2 (bloc 103) et requiert de l'opérateur l'introduction, à la station de chargement auxiliaire 3, d'un plateau d'essai 33a (bloc 104). A cette fin, on peut prévoir à la station 3 des moyens de signalisation adéquats, non représentés. L'opérateur introduit alors dans l'installation un plateau d'essai 33a contenant des dispositifs de commande de l'opération. Le plateau d'essai 33a est constitué par deux boîtes d'essai 31a, 31b (figure 5), la boîte 31a disposée en premier dans le sens de la marche étant vide et la deuxième boîte 31b contenant des moyens de mesure de l'irradiation, tels qu'un calorimètre 96. Ensuite, sur commande de l'opérateur ou automatiquement, après que le code du plateau a été lu par le lecteur 45 (bloc 105), après que l'opérateur a enfoncé une touche de code d'identification du dispositif de commande et de contrôle (bloc 106) et après que le code du plateau et le code introduit manuellement ont été associé (bloc 107), les dispositifs de blocage 58a (bloc 108) sont désactivés par l'avancement du plateau introduit à la main dans la station auxiliaire 3 et du plateau d'essai 33a et des plateaux 33 chargés précédemment à la station de chargement principale 2.

Lors du cheminement le long des moyens de convoyage, les lecteurs 51 et 52 (et, éventuellement, d'autres lecteurs non représentés) lisent le code d'identification des plateaux pour suivre le cheminement des plateaux et des produits transportés sur tout le trajet extérieur au caisson (blocs 110, 111). Les informations fournies par ces lecteurs, les données relatives à la synchronisation du moteur 55 et, éventuellement, les données fournies par les dispositifs de fin de course mécaniques 61, 65 pour ce qui concerne l'intérieur du caisson 6 (bloc 112), sont utilisées par l'unité de commande 42 pour localiser ou identifier de façon précise, à tout moment, la position exacte de chaque plateau 33 à l'intérieur du caisson. En cas d'anomalie ou lorsque le fonctionnement de l'installation est interrompu, les boîtes de produits qui ont été traitées totalement, celles qui l'ont été partiellement et celles dont l'état n'est pas parfaitement connu peuvent ainsi être repérées. En outre, en cas d'anomalie grave, l'unité de commande et de contrôle 42 peut directement arrêter le traitement en stoppant l'accélérateur 70 (bloc 114), en envoyant les signaux d'erreur correspondants à l'opérateur (115) et en déchargeant les plateaux 33 présents sur les moyens de convoyage 8 (116).

Comme cela a été mentionné plus haut, le convoyeur 62 de la section de convoyage 14 est plus rapide que le convoyeur 63 (dont les deux parties 63a et 63b se déplacent à la même vitesse). Le convoyeur 62 éloigne rapidement du convoyeur 60 les plateaux 33 apportés par ce convoyeur et provoque le regroupement des plateaux 33 dans la direction de cheminement de façon à réduire la distance entre deux plateaux successifs. En outre, le début des rails de guidage 67 des plateaux (non visible sur les figures) et la position des organes d'entraînement 84 des chaînes 72 sont étudiés de manière que les organes d'entraînement ne viennent en contact du cadre inférieur 36 de chaque plateau 33 que lorsque celui-ci est arrivé à la fin de la section de convoyage 13, ceci afin d'éviter une rotation du plateau 33.

Les convoyeurs 62 et 63a sont synchronisés exactement de manière que, lorsque un plateau 33 atteint le convoyeur 63a, une paire d'organes d'entraînement 84 des chaînes 73 s'engage dans le cadre inférieur 36 du plateau 33, après avoir pivoté de la position rétractée représentée sur la figure 13 à la position saillante représentée sur la figure 14. Le pivotement des organes d'entraînement 84 a pour effet de leur communiquer un mouvement horizontal qui s'ajoute au déplacement résultant de l'entraînement par les chaînes 73; ce pivotement provoque donc une légère augmentation de la vitesse des organes 84 par rapport à la vitesse du convoyeur 63a. En conséquence, au moment où un plateau 33 est entraîné par le convoyeur 63a, ce plateau subit une légère poussée vers l'avant, ce qui diminue encore la distance qui le sépare du plateau précédent (de l'ordre de quelques millimètres) sans que le plateau qui suit ne heurte celui qui le précède. Les guides rectilignes 90 associés au convoyeur 62 ne sont pas aussi long que ce convoyeur de sorte qu'un plateau 33 est libéré par le convoyeur 62 avant d'être entraîné par le convoyeur 63a, et il ne subit aucune poussée en dépit de la différence de vitesse des deux convoyeurs.

Dans la partie centrale de la section de convoyage 14, les plateaux 33 sont très rapprochés et cheminent à une vitesse constante, ce qui assure une irradiation régulière dans la station d'irradiation 7. Dans cette zone, les convoyeurs 63a, 63b se comportent comme un convoyeur unique puisque leurs vitesses sont égales, et grâce à l'interstice qui les sépare ils ne sont pas endommagés par l'irradiation.

Lorsqu'un plateau d'essai 33a a été introduit sur les moyens de convoyage à la station de chargement auxiliaire 3 (bloc 120), l'unité de commande et de contrôle 42 détermine le moment où ce plateau 33a atteindra la station d'irradiation 7, mémorise les paramètres de traitement correspondants (bloc 121) et commande la modification des paramètres d'irradiation pour les adapter au nouveau produit à traiter (bloc 122). De la sorte, lorsque le plateau d'essai 33a atteint la station d'irradiation 7, l'accélérateur 70 règle les paramètres d'irradiation au moment du passage de la première boîte 31a (vide), et, lors du passage de la seconde boîte 31b, le calorimètre 96 qu'elle contient mesure la dose reçue. Le plateau d'essais 33a est déchargé ensuite à la station de déchargement auxiliaire 5 et on vérifie si la dose reçue correspond à la valeur commandée (blocs 123, 124, 125). Un lecteur de code peut être éventuellement prévu à la station de déchargement auxiliaire 5 pour permettre de confirmer l'acheminement correct des plateaux d'essai.

L'unité de commande et de contrôle 42 continue, en outre, à contrôler la vitesse d'acheminement des plateaux dans la station d'irradiation 7 par un signal envoyé à la dynamo tachymétrique 56 (bloc 126) et, s'il se produit une anomalie dans la vitesse du moteur 55 qui peut être compensée par un ajustement de la dose irradiée, elle commande la modification des paramètres de fonctionnement de l'accélérateur 70 (bloc 127).

Après avoir été irradiés dans la station 7, les plateaux 33 sont transférés du convoyeur 63b sur le convoyeur 64, dont la vitesse a été choisie supérieure à celle des convoyeurs 63a, 63b pour que plateaux traités 33 soient acheminés rapidement vers l'extrémité de la section de convoyage 14. Dès qu'un plateau 33 atteint le détecteur de fin de course 65, ce dernier actionne le convoyeur à rouleaux 66 qui entraîne le plateau 33 hors de la section 14, sans en modifier l'orientation. Le convoyeur 66 achemine les plateaux 33 jusqu'au transporteur vertical 92 qui les prélève, l'un après l'autre, sur le convoyeur 66, les fait descendre au niveau du convoyeur 97, et les transfère sur ce dernier.

Les plateaux 33 chargés de boîtes 31 irradiées sont ainsi acheminées sur les sections de convoyage 17-20 au dessous des sections de convoyage pour les plateaux chargés de boîtes 31 de produits à traiter. Dans le cas où ils ne contiennent pas de boîtes d'essai 31a, 31b, les plateaux sont acheminés au moyens des convoyeurs 47, 47b, 47c vers la station de déchargement principale 4 (bloc 128). Là, le lecteur 38 lit l'étiquette 37 du plateau 33 et communique à l'unité de commande et de traitement 42 l'arrivée correcte des plateaux 33 à la sortie de l'installation.

L'installation décrite plus haut présente les avantages suivants. Elle est fiable et efficace, notamment grâce à ce que, dans la partie centrale de la section 14, les espaces entre les plateaux sont réduits à quelques millimètres (ce qui réduit donc pratiquement à zéro le temps de fonctionnement à vide de l'accélérateur). Son rendement élevé est dû en outre à ce qu'elle permet de modifier les paramètres de l'irradiation requis par des produits différents sans avoir à arrêter l'installation.

La vitesse d'acheminement constante, en particulier dans la section de convoyage 14, garantit l'uniformité de l'irradiation reçue par les produits et permet de traiter des produits délicats, tels que des lignes à sang en PVC, pour lesquels l'écart est réduit entre les doses maximales et minimales admissibles pour assurer la stérilisation et éviter détérioration des produits. Le contrôle de la totalité du convoyage, y compris à l'intérieur du caisson (au moyen de la dynamo tachymétrique associée au moteur) contribue à assurer la fiabilité de l'installation et permet de compenser de faibles irrégularités de vitesse, comme décrit plus haut. Par ailleurs, l'unité de commande et de contrôle est en mesure de détecter toute anomalie susceptible d'affecter l'efficacité de la stérilisation, d'interrompre le traitement, et distinguer les boîtes qui ont été traitées de celles qui ne l'ont pas été ou insuffisamment.

La superposition de plusieurs sections de convoyage des produits à traiter et des produits traités permet de limiter l'espace occupé par l'installation; l'utilisation d'un transporteur vertical permet de réduire l'espace occupé à l'intérieur du caisson, dont le coût de fabrication est élevé

L'association des boîtes de produits 31 aux plateaux 33 et le contrôle, effectué uniquement sur les plateaux à l'intérieur de l'installation, permettent de réduire les contrôles périodiques du cheminement des produits, même lorsque les conditions ambiantes ne favorisent pas la lecture des étiquettes. En fait, les codes imprimés sur les plateaux peuvent avoir une meilleure qualité d'impression (ce qui facilite ainsi la lecture) que les codes imprimés sur le carton des boîtes de produits. On évite ainsi les arrêts de l'installation résultant du problème de lecture des codes. La présence des détecteurs de fin de course mécaniques dans la zone critique interne 27 et l'entraînement des plateaux par un système d'accrochage qui rend les plateaux 33 solidaires des convoyeurs garantissent, d'une part, la possibilité de contrôler le convoyage, même dans la zone critique 27 et, d'autre part, la sécurité du convoyage des plateaux. En outre, l'identification de tous les plateaux et des boîtes qu'ils supportent est assurée, même quand l'accélérateur est éteint, sans interrompre le convoyage des plateaux. Par ailleurs, l'inaccessibilité de l'ensemble des moyens de convoyage grâce à la cage en treillis 43 et au caisson 6 garantit l'impossibilité d'une modification des associations boîtes/plateaux établies à l'entrée dans l'installation.

L'utilisation de matériaux résistant aux radiations ionisantes et l'absence d'appareillage électronique à l'intérieur de la zone critique 27 garantissent le fonctionnement adéquat de l'installation et sa longue durée de service. Grâce à toutes les caractéristiques avantageuses qui viennent d'être mentionnées, il est possible de réduire le coût unitaire de traitement des produits et de faire subir une stérilisation par irradiation par rayons bêta à des produits ayant un faible coût unitaire.

## Revendications

1. Installation de stérilisation (1) de produits médicaux (31) par irradiation, comprenant:
- au moins une station de chargement (2) de produits,
- au moins une station de déchargement (4) de produits,
- une station d'irradiation (7) située à l'intérieur d'un caisson (6) ayant des parois aptes à arrêter les rayonnements stérilisants,
- des moyens de convoyage (10-21) pour acheminer les produits (31) à stériliser de la station de chargement (2) à la station d'irradiation (7) et les produits (31) stérilisés de la station d'irradiation (7) à la station de déchargement (4), les moyens de convoyage comprenant des moyens de regroupement (62, 63) pour regrouper les produits (31) à stériliser à proximité de la station d'irradiation (7), les moyens de regroupement comprenant un premier convoyeur (62) et un deuxième convoyeur (63), le deuxième convoyeur (63) étant situé immédiatement en aval du premier convoyeur (62) par rapport à la direction de convoyage des produits (31) et ayant une vitesse de convoyage inférieure à la vitesse de convoyage du premier convoyeur (62).

2. Installation selon la revendication 1 , **caractérisée en ce que** le premier et le deuxième convoyeurs (62, 63) comprennent des organes d'entraînement (84) rétractables pour accrocher les produits (31) et assujettir rigoureusement leur déplacement à celui du convoyeur (62, 63) correspondant.

3. Installation selon une des revendications 1 et 2, **caractérisée en ce que** le deuxième convoyeur (63) comprend deux demi convoyeurs (63a, 63b) séparés par un interstice disposé dans une zone vers laquelle la station d'irradiation (7) émet les rayonnements stérilisants.

4. Installation selon une des revendications 2 à 3, **caractérisée en ce qu'**elle comprend un troisième convoyeur (64) situé immédiatement en aval du deuxième convoyeur (63) par rapport à la direction de convoyage des produits (31), le troisième convoyeur (64) ayant une vitesse de convoyage supérieure à la vitesse de convoyage du deuxième convoyeur (63).

5. Installation selon une des revendications 3 et 4, **caractérisée en ce que** au moins le premier et le deuxième convoyeurs (62, 63) sont des convoyeurs à chaînes comprenant chacun une paire de chaînes (72, 73, 74) et **en ce que** les organes d'entraînement (84) comprennent chacun un doigt et sont montés pivotants sur les chaînes pour pouvoir pivoter entre une position rétractée où le doigt ne peut pas accrocher de produit (31) et une position saillante où le doigt peut accrocher un produit (31).

6. Installation selon l'une des revendications 1 à 5, **caractérisée en ce que** les moyens de convoyage (10-21) comprennent des sections de convoyage (11-13 et 18-20) superposées, les sections de convoyage (11-13) situées à un premier niveau acheminant les produits (31) à stériliser de la station de chargement (2) à la station d'irradiation (7) et les sections de convoyage (18-20) situées à un second niveau acheminant les produits (31) stérilisés de la station d'irradiation (7) à la station de déchargement (4).

7. Installation selon la revendication 6, caractérisée en ce les moyens de convoyage (10-21) comprennent un transporteur vertical (92) pour transférer les produits (31) d'un niveau de convoyage à l'autre niveau de convoyage.

8. Installation selon l'une des revendications 1 à 7, **caractérisée en ce qu'**elle comprend des moyens de détection (38, 40, 41, 44, 45, 51, 52, 61, 65) de produits (31), disposés le long des moyens de convoyage (10-21), et des moyens de commande et de contrôle (42) reliés aux moyens de détection pour suivre le cheminement des produits (31) sur les moyens de convoyage (10-21).

9. Installation selon la revendication 8, **caractérisée en ce qu'**elle comprend une pluralité de plateaux (33) destinés à supporter les produits (31) sur les moyens de convoyage (10-21), chaque plateau (33) étant muni d'un code d'identification (37), les moyens de détection de produits (31) comprenant des moyens de lecture de code (38, 40, 41, 44, 45, 51, 52) aptes à lire le code (37) apposé sur chaque plateau (33).

10. Installation selon la revendication 9, **caractérisée en ce que** les moyens de détection de produits (31) comprennent des moyens de lecture de code (38, 40, 41, 44, 45, 51, 52) aptes à lire un code d'identification (32) apposé sur chacun des produits (31) disposé sur chaque plateau (33) et **en ce que** l'unité de commande et de contrôle (42) est prévue pour associer et mettre en mémoire le code d'identification (32) de chaque produit (31) et le code d'identification (37) du plateau qui supporte ce produit (31).

11. Installation selon une des revendication 9 et 10 à 11, **caractérisée en ce que** les moyens de détection de produits (31) comprennent des moyens de détection de fin de course mécaniques (61, 65) disposés à l'intérieur du caisson (6) pour détecter la position des plateaux (33) à l'intérieur du caisson (6) et transmettre un signal correspondant à l'unité de commande et de contrôle (42).

12. Installation selon une des revendication 8 à 11, **caractérisée en ce que** les moyens de détection de produits (31) comprennent des moyens de lecture de code (40, 41, 44) disposés à proximité de la station de chargement (2), et **en ce que** les moyens de commande et de contrôle (42) sont prévus pour comparer chaque produit (31) introduit dans la station de chargement (5) au produit introduit précédemment et pour commander le déclenchement de moyens d'avertissement lorsque ces produits ne sont pas de même type.

13. Installation selon une des revendication 8 à 12, **caractérisée en ce qu'**elle comporte des moyens de mesure (56) de la vitesse du deuxième convoyeur (63) reliés aux moyens de commande et de contrôle (42), et **en ce que** les moyens de commande et de contrôle (42) sont prévus pour ajuster l'irradiation émise par la station d'irradiation en fonction des variations de la vitesse du convoyeur (63) de façon que les produits (31) reçoivent une dose d'irradiation prédéterminée .

14. Installation selon une des revendications 1 à 13, **caractérisée en ce qu'**elle comprend une cage en treillis (43) entourant les moyens de convoyage (10-21) à l'extérieur du caisson (6).

## Patentansprüche

1. Einrichtung zur Sterilisierung (1) medizinischer Produkte (31) durch Bestrahlung mit:
- zumindest einer Beschickungsstation (2) für Produkte,
- zumindest einer Entladestation (4) für Produkte,
- einer Bestrahlungsstation (7), die im Inneren eines Kastens (6) mit Wänden liegt, die in der Lage sind, die sterilisierende Strahlung zu stoppen,
- Fördermitteln (10-21), um die zu sterilisierenden Produkte (31) von der Beschickungsstation (2) zur Bestrahlungsstation (7) und die sterilisierten Produkte von der Bestrahlungsstation (7) zur Entladestation (4) zu befördern, wobei die Fördermittel Umschichtmittel (62, 63) umfassen, um die zu sterilisierenden Produkte (31) in der Nähe der Bestrahlungsstation (7) umzuschichten, wobei die Umschichtmittel eine erste Fördereinrichtung (62) und eine zweite Fördereinrichtung (63) umfassen, wobei die zweite Fördereinrichtung (63) relativ zur Förderrichtung der Produkte (31) unmittelbar stromabwärts von der ersten Fördereinrichtung (62) liegt und eine Fördergeschwindigkeit aufweist, die geringer als die Fördergeschwindigkeit der ersten Fördereinrichtung (62) ist.

2. Einrichtung nach Anspruch 1, **dadurch gekennzeichnet, dass** die erste und die zweite Fördereinrichtung (62, 63) einziehbare Mitnahmeglieder (84) umfassen, um die Produkte (31) anzukoppeln und ihrer Bewegung streng diejenige der entsprechenden Fördereinrichtung (62, 63) aufzuzwingen.

3. Einrichtung nach einem der Ansprüche 1 und 2, **dadurch gekennzeichnet, dass** die zweite Fördereinrichtung (63) zwei Halbfördereinrichtungen (63a, 63b) umfasst, die durch einen Zwischenraum getrennt sind, der in einem Bereich angeordnet ist, zu dem die Bestrahlungsstation (7) die sterilisierenden Strahlen emittiert.

4. Einrichtung nach einem der Ansprüche 2 und 3, **dadurch gekennzeichnet, dass** sie eine dritte Fördereinrichtung (64) umfasst, die relativ zur Förderrichtung der Produkte (31) unmittelbar stromabwärts von der zweiten Fördereinrichtung (63) liegt, wobei die dritte Fördereinrichtung (64) eine Fördergeschwindigkeit aufweist, die größer als die Fördergeschwindigkeit der zweiten Fördereinrichtung (63) ist.

5. Einrichtung nach einem der Ansprüche 3 und 4, **dadurch gekennzeichnet, dass** zumindest die erste und die zweite Fördereinrichtung (62, 63) Kettenfördereinrichtungen sind, die jeweils ein Kettenpaar (72, 73, 74) umfassen, und dass die Mitnahmeglieder (84) jeweils einen Finger umfassen und schwenkbar an den Ketten angebracht sind, um zwischen einer eingezogenen Position, in der der Finger kein Produkt (31) ankoppeln kann, und einer hervorstehenden Position schwenken zu können, in der der Finger ein Produkt (31) ankoppeln kann.

6. Einrichtung nach einem der Ansprüche 1 bis 5, **dadurch gekennzeichnet, dass** die Fördermittel (10-21) übereinander liegende Förderabschnitte (11-13 und 18-20) umfassen, wobei die auf einem ersten Niveau liegenden Förderabschnitte (11-13) die zu sterilisierenden Produkte (31) von der Beschickungsstation (2) zu der Bestrahlungsstation (7) bringen und die auf einem zweiten Niveau liegenden Förderabschnitte (18-20) die sterilisierten Produkte (31) von der Bestrahlungsstation (7) zu der Entladestation (4) bringen.

7. Einrichtung nach Anspruch 6, **dadurch gekennzeichnet, dass** die Fördermittel (10-21) eine vertikale Transporteinrichtung (92) umfassen, um die Produkte (31) von dem einen Förderniveau auf das andere Förderniveau zu übertragen.

8. Einrichtung nach einem der Ansprüche 1 bis 7, **dadurch gekennzeichnet, dass** sie Erfassungsmittel (38, 40, 41, 44, 45, 51, 52, 61, 65) für Produkte (31) umfasst, die längs der Fördermittel (10-21) angeordnet sind, und Steuer- und Kontrollmittel (42), die mit den Erfassungsmitteln verbunden sind, um die Beförderung der Produkte (31) auf den Fördermitteln (10-21) zu verfolgen.

9. Einrichtung nach Anspruch 8, **dadurch gekennzeichnet, dass** sie eine Vielzahl von Tabletts (33) umfasst, die dazu bestimmt sind, die Produkte (31) auf den Fördermitteln (10-21) zu tragen, wobei jedes Tablett (33) mit einem Identifizierungskode (37) versehen ist, wobei die Erfassungsmittel für Produkte (31) Kodelesemittel (38, 40, 41, 44, 45, 51, 52) umfassen, die in der Lage sind, den an jedem Tablett (33) angebrachten Kode (37) zu lesen.

10. Einrichtung nach Anspruch 9, **dadurch gekennzeichnet, dass** die Erfassungsmittel für Produkte (31) Mittel zum Lesen des Kodes (38, 40, 41, 44, 45, 51, 52) umfassen, die in der Lage sind, einen an jedem der auf jedem Tablett (33) angeordneten Produkte (31) angebrachten Identifizierungskode (32) zu lesen, und dass die Steuer- und Kontrolleinheit (42) dafür vorgesehen ist, den Identifizierungskode (32) jedes Produkts (31) und den Identifizierungskode (37) des Tabletts, das dieses Produkt (31) trägt, zu verknüpfen und zu speichern.

11. Einrichtung nach einem der Ansprüche 9 und 10, **dadurch gekennzeichnet, dass** die Erfassungsmittel für Produkte (31) mechanische Mittel zur Erfassung des Bahnendes (61, 65) umfassen, die im Inneren des Kastens (6) angeordnet sind, um die Position der Tabletts (33) im Inneren des Kastens (6) zu erfassen und ein entsprechendes Signal zu der Steuer- und Kontrolleinheit (42) zu übertragen.

12. Einrichtung nach einem der Ansprüche 8 bis 11, **dadurch gekennzeichnet, dass** die Erfassungsmittel für Produkte (31) Kodelesemittel (40, 41, 44) umfassen, die in der Nähe der Beschickungsstation (2) angeordnet sind, und dass die Steuer- und Kontrollmittel (42) dafür vorgesehen sind, jedes in die Beschickungsstation (5) eingeführte Produkt (31) mit dem zuvor eingeführten Produkt zu vergleichen und die Auslösung von Warnmitteln zu steuern, wenn diese Produkte nicht von der gleichen Art sind.

13. Einrichtung nach einem der Ansprüche 8 bis 12, **dadurch gekennzeichnet, dass** sie Messmittel (56) für die Geschwindigkeit der zweiten Fördereinrichtung (63) umfasst, die mit den Steuer- und Kontrollmitteln (42) verbunden sind, und dass die Steuer- und Kontrollmittel (42) dafür vorgesehen sind, die von der Bestrahlungsstation abgegebene Bestrahlung in Abhängigkeit von einer Geschwindigkeitsänderung der Fördereinrichtung (63) anzupassen, so dass die Produkte (31) eine vorbestimmte Bestrahlungsdosis empfangen.

14. Einrichtung nach einem der Ansprüche 1 bis 13, **dadurch gekennzeichnet, dass** sie einen Drahtgitterkäfig (43) umfasst, der die Fördermittel (10-21) außerhalb des Kastens (6) umgibt.

## Claims

1. Installation (1) for the sterilization of medical products (31) by irradiation comprising:
- at least one product loading station (2),
- at least one product unloading station (4),
- an irradiation station (7) located inside a containment (6) having walls capable of stopping sterilizing radiation,
- conveyance means (10-21) for conveying the products (31) to be sterilized from the loading station (2) to the irradiation station (7) and the sterilized products (31) from the irradiation station (7) to the unloading station (4), the conveyance means comprising grouping means (62, 63) for grouping the products (31) to be sterilized in the vicinity of the irradiation station (7), the grouping means comprising a first conveyor (62) and a second conveyor (63), the second conveyor (63) being located immediately downstream of the first conveyor (62) in relation to the direction of conveyance of the products (31) and having a conveying speed lower than the conveying speed of the first conveyor (62).

2. Installation according to Claim 1, **characterized in that** the first and second conveyors (62, 63) comprise retractable drive members (84) for catching the products (31) and strictly subjecting their displacement to that of the corresponding conveyor (62, 63).

3. Installation according to one of Claims 1 and 2, **characterized in that** the second conveyor (63) comprises two half-conveyors (63a, 63b) separated by a gap located in a zone towards which the irradiation station (7) emits the sterilizing radiation.

4. Installation according to one of Claims 2 to 3, **characterized in that** it comprises a third conveyor (64) located immediately downstream of the second conveyor (63) in relation to the direction of conveyance of the products (31), the third conveyor (64) having a conveying speed higher than the conveying speed of the second conveyor (63).

5. Installation according to one of Claims 3 to 4, **characterized in that** at least the first and second conveyors (62, 63) are chain conveyors, each comprising a pair of chains (72, 73, 74) and **in that** the drive members (84) each comprise a finger and are mounted pivotably on the chains; so as to be capable of pivoting between a retracted position,. in which the finger cannot catch any product (31), and a projecting position, in which the finger can catch a product (31).

6. Installation according to one of Claims 1 to 5, **characterized in that** the conveyance means (10-21) comprise superposed conveyance sections (11-13 and 18-20), the conveyance sections (11-13) located at a first level conveying the products (31) to be sterilized from the loading station (2) to the irradiation station (7), and the conveyance sections (18-20) located at a second level conveying the sterilized products (31) from the irradiation station (7) to the unloading station (4).

7. Installation according to Claim 6, **characterized in that** the conveyance means (10-21) comprise a vertical transporter (92) for transferring the products (31) from one conveyance level to the other conveyance level.

8. Installation according to one of Claims 1 to 7, **characterized in that** it comprises means (38, 40, 41, 44, 45, 51, 52, 61, 65) for the detection of products (31), the said detection means being arranged along the conveyance means (10-21), and control and monitoring means (42) connected to the detection means in order to follow the travel of the products (31) on the conveyance means (10-21).

9. Installation according to Claim 8, **characterized in that** it comprises a plurality of trays (33) intended for supporting the products (31) on the conveyance means (10-21), each tray (33) being provided with an identification code (37), the means for the detection of. products (31) comprising code-reading means (38, 40, 41, 44, 45, 51, 52) capable of reading the code (37) affixed to each tray (33).

10. Installation according to Claim 9, **characterized in that** the means for the detection of products (31) comprise code-reading means (38, 40, 41, 44, 45, 51, 52) capable of reading an identification code (32) affixed to each of the products (31) which is arranged on each tray (33), and **in that** the control and monitoring unit (42) is provided for combining and storing the identification code (32) of each product (31) and the identification code (37) of the tray which supports this product (31).

11. Installation according to one of Claims 9 to 10, **characterized in that** the means for the detection of products (31) comprise mechanical travel-limit detection means (61, 65) arranged inside the containment (6), in order to detect the position of the trays (33) inside the containment (6) and transmit a corresponding signal to the control and monitoring unit (42).

12. Installation according to one of Claims 8 to 11, **characterized in that** the means for the detection of products (31) comprise code-reading means (40, 41, 44) arranged in the vicinity of the loading station (2), and **in that** the control and monitoring means (42) are provided for comparing each product (31) introduced in the loading station (5) with the product previously introduced and for controlling the triggering of warning means when these products are not of the same type.

13. Installation according to one of Claims 8 to 12, **characterized in that** it comprises means (56) for measuring the speed of the second conveyor (63), which are connected to the control and monitoring means (42), and **in that** the control and monitoring means (42) are provided for adjusting the irradiation emitted by the irradiation station as a function of the variations in the speed of the conveyor (63), in such a way that the products (31) receive a predetermined irradiation dose.

14. Installation according to one of Claims 1 to 13, **characterized in that** it comprises a trelliswork cage (43) surrounding the conveyance means (10-21) outside the containment (6).
